# EUROPEAN PATENT APPLICATION

(11) **EP 4 159 722 A1**
(43) Date of publication of application: **05.04.2023**
(21) Application number: 21812621.7
(22) Date of filing: 26.05.2021
(51) Int. Cl.: C07D 217/02, C07D 217/18, C07D 217/20, A61K 31/472, A61K 31/4741, A61P 3/04, A61P 3/10

(54) **METHOD FOR PREPARING GLP-1 RECEPTOR AGONIST**

(30) Priority: 28.05.2020 CN 202010481869
(71) Applicant: Hangzhou Zhongmeihuadong Pharmaceutical Co., Ltd., GongShu District HangZhou, Zhejiang 310011 (CN)
(72) Inventor: HU, Fan, HangZhou, Zhejiang 310011 (CN); ZHOU, Yubao, HangZhou, Zhejiang 310011 (CN); FANG, Li, HangZhou, Zhejiang 310011 (CN); HU, Haiwen, HangZhou, Zhejiang 310011 (CN); XU, Zhongjun, HangZhou, Zhejiang 310011 (CN)
(74) Representative: Mewburn Ellis LLP
(86) International application number: PCT/CN2021/095963
(87) International publication number: WO 2021/238962

(57) **Abstract**

The present invention relates to a method for preparing GLP-1 receptor agonist, specifically relates to an industrialized method for preparing a GLP-1 receptor agonist (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridi n-4-yl)phenyl)propanoic acid dihydrochloride (compound I). The method utilizes compound 1 as the starting material, which undergoes nucleophilic addition, hydrolysis, reduction ammoniation, cyclization, amide condensation, hydrolysis, and salt forming reaction to give compound I. The method for preparing compound I has a total yield of more than 35%, with purity of compound I more than 98%.

## Description

### Technical Field

The invention belongs to the technical field of drug synthesis, and particularly relates to a method for preparing (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propanoic acid dihydrochloride (compound I).

### Background of the Invention

(S)-2-(3S,8S)-3-(4-(3,4-dichlorobenzyloxy)phenyl-7-((S)-1-phenylpropyl)-2,3,6,7,8,9-hexahydro-[ 1,4]-dioxino[2,3-g]isoquinolin-8-ylformylamino)-3-(4-(2,3-dimethylpyridin-4-yl)phenyl)propanoic acid dihydrochloride, is an oral, non-peptide glucagon-like peptide 1 (GLP-1) receptor agonist with high selectivity, which was world-firstly developed by vTv Therapeutics LLC (US) and had completed Phase II clinical study. The clinical study demonstrated that, compound I could significantly reduce the level of glycosylated hemoglobin in diabetic patients, and were well-tolerated. (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propanoic acid dihydrochloride has a molecular formula of C₅₀H₄₉Cl₄N₃O₆, a molecular weight of 929.76, and the following chemical structure:

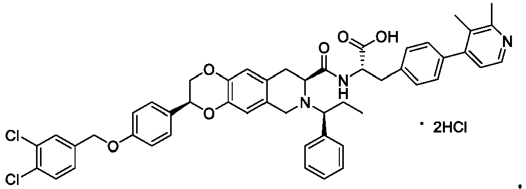

A patent for invention CN102378574B discloses a method for preparing compound I free base, which utilizes 4-hydroxyacetophenone as the starting material and comprises 14 steps including nucleophilic substitution, bromination, asymmetric reduction, condensation, hydrolysis, etc.

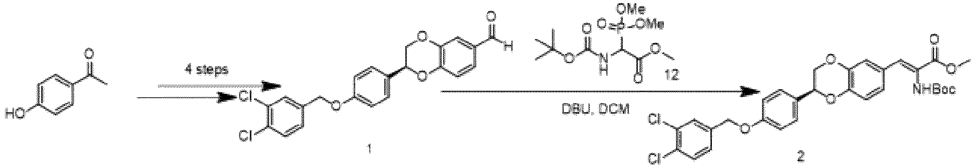

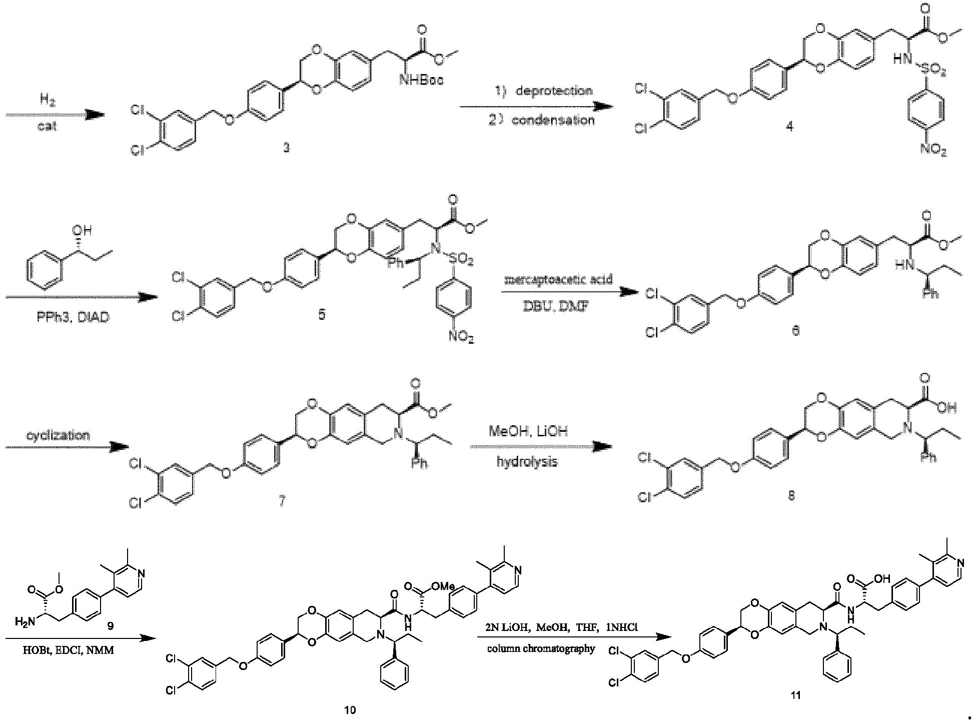

However, the preparation method includes many steps, requires multiple column chromatographic separations, and has a total yield of only 5%, which makes it difficult to apply to industrial production. Thus, it is necessary to redevelop a synthetic route suitable for industrial production.

### Summary of the Invention

The present invention provides a method for preparing (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4 -yl)phenyl)propanoic acid dihydrochloride (compound I). Compared with the prior art, the method has less steps and increased yield, and adopts multi-step continuous feeding reaction, which does not require column chromatographic separation and is suitable for industrial production.

The preparation method of the invention is as follows: compound 1 is utilized as the starting material, and undergoes nucleophilic addition, hydrolysis, reduction ammoniation, cyclization, amide condensation, hydrolysis, and salt forming reaction to give compound I.

According to the invention, the specific preparation method comprises the following steps:
a) Reacting compound 1 with compound 2 via addition reaction to give compound VII; 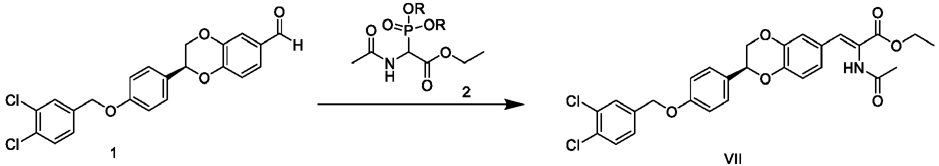
b) Subjecting compound VII to hydrolysis to give compound VI; 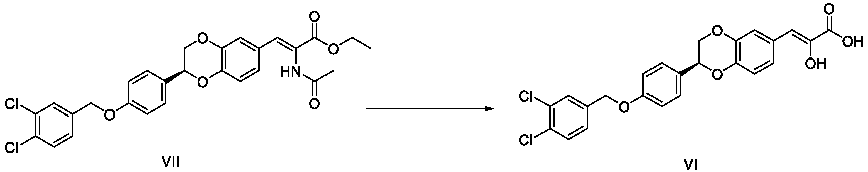
c) Reacting compound VI with S-phenylpropylamine via ammoniation reduction to give compound V; 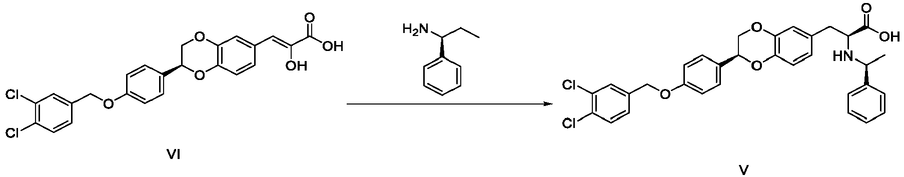
d) Reacting compound V with paraformaldehyde via cyclization to give compound IV; 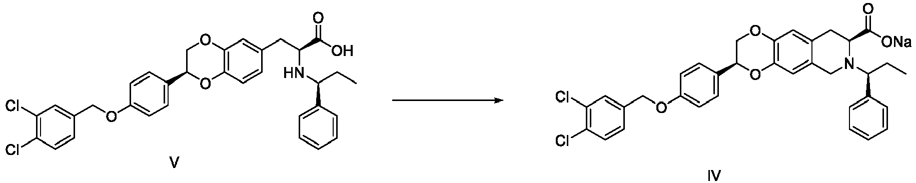
e) Reacting compound IV with compound 9 via condensation to give compound III; 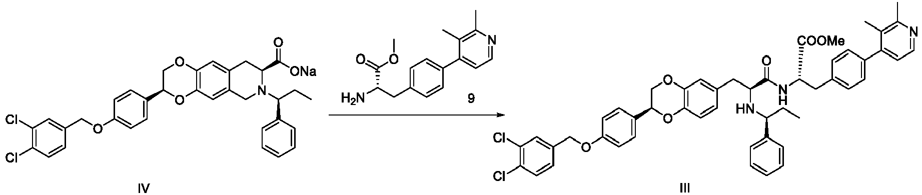
f) Subjecting compound III to hydrolysis under alkaline condition, and then adjusting pH to acidic condition, to give compound II; 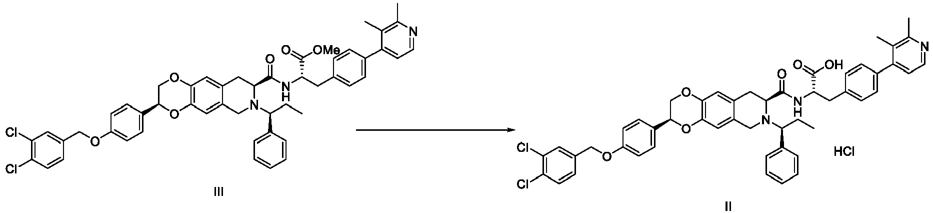
g) Reacting compound II with hydrochloric acid solution to give compound I;

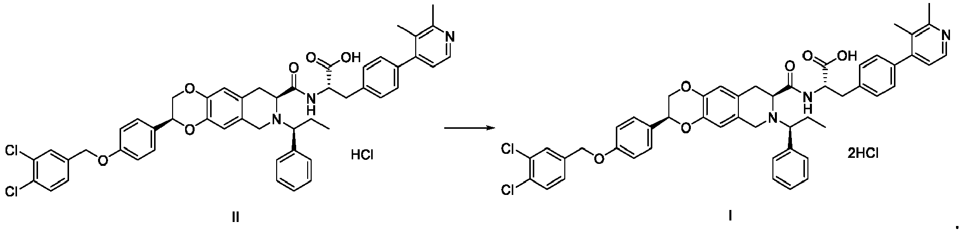

As a specific embodiment, in step a), the reaction solvent is one, two or more selected from the group consisting of dichloromethane, toluene, tetrahydrofuran, and 2-methyltetrahydrofuran, and preferably 2-methyltetrahydrofuran.

As a specific embodiment, in step a), the R group of Compound 2 is selected from C1^{~}C10 alkane, C3^{~}C8 cycloalkane, benzyl, aryl and heteroaryl, preferably methyl.

As a specific embodiment, step a) is carried out under alkaline condition using organic base as the alkaline reagent, wherein the organic base is one, two or more selected from the group consisting of DBU, DMAP, triethylamine, isopropylamine, dibutylamine, piperazine, piperidine, and morpholine, and preferably DMAP.

As a specific embodiment, compound 1, 2 and the alkaline reagent are fed at a molar ratio in the range of 1:1.05:1.5^{~}1:2:3.

As a specific embodiment, the workup mode of step a) is as follows: after the completion of the reaction, an aqueous solution of ammonium chloride is added to the reaction, and the reaction mixture is stirred and washed; the aqueous layer is separated and removed out, and the organic layer is washed with saturated brine and then concentrated; methanol/water is added to the concentrated solution and stirred to crystalize, and the precipitated crystals are filtered and dried to give compound VII.

As a specific embodiment, in step b), the reaction solvent is one, two or more selected from the group consisting of 1,4-dioxane, N-methylpyrrolidone, dimethyl sulfoxide, dimethylacetamide, and dimethylformamide, and preferably N-methylpyrrolidone.

As a specific embodiment, step b) is carried out under acidic condition using acidic aqueous solution as the acidic reagent, wherein the acid is one, two or more selected from the group consisting of hydrochloric acid, sulfuric acid, and phosphoric acid, and preferably hydrochloric acid.

As a specific embodiment, compound VII and the acidic aqueous solution are fed at a molar ratio in the range of 1:10^{~}1:13, and the concentration of the acidic aqueous solution is 2 mol/L^{~}6 mol/L.

As a specific embodiment, the workup mode of step b) is as follows: after the completion of the reaction, the temperature is lowered and water is added and stirred to crystalize; wet product is obtained after filtration, and then the wet product is slurried with methanol/dichloromethane; and compound VI is obtained after filtration and drying.

As a specific embodiment, in step c), the reaction solvent is one, two or more selected from the group consisting of dioxane, N-methylpyrrolidone, dimethyl sulfoxide, toluene, dimethylformamide, and dimethylacetamide, and preferably a mixture of toluene and dimethylacetamide.

As a specific embodiment, step c) is carried out under alkaline condition using organic base as the alkaline reagent, wherein the organic base is triethylamine or/and isopropylamine.

As a specific embodiment, in step c), the compound VI, S-phenylpropylamine and the organic base are fed at a molar ratio in the range of 1:1.05:1.05^{~}1:1.1:3.

As a specific embodiment, in step c), a reducing agent is added to the reaction, wherein the reducing agent is NaBH(OCOR)₃, with R being C₆^{~}C₁₂ alkyl, and preferably C₇-C₉ alkyl. The molar amount of the reducing agent is 1^{~}3 times to that of compound VI, and preferably 1.5^{~}2 times.

As a specific embodiment, the workup mode of step c) is as follows: after the completion of the reaction, partial solvent is removed out by concentration, and then methanol is added and stirred to crystalize.

As a specific embodiment, in step d), the reaction solvent is one, two or more selected from the group consisting of toluene, xylene, chlorobenzene, ethyl acetate, isopropyl acetate, propyl acetate, and butyl acetate, and preferably a mixture of chlorobenzene and isopropyl acetate.

As a specific embodiment, in step d), acid catalyst is added to the reaction, wherein the acid catalyst is one, two or more selected from the group consisting of trifluoroacetic acid, concentrated sulfuric acid, hydrochloric acid, and trifluoromethanesulfonic acid, and preferably trifluoromethanesulfonic acid.

As a specific embodiment, step d) is carried out at a temperature in the range of 20^{~}50°C, and preferably in the range of 30^{~}40°C.

As a specific embodiment, the workup mode of step d) is as follows: the temperature is lowered, and a sodium hydroxide solution of 1^{~}4 mol/L is added at a weight amount of 8^{~}11 times to that of compound V; after stirring and extraction, organic layer is separated and then washed with saturated brine; a high boiling solvent is added, and then solvent of step d) is removed out by stirring and concentration; the concentrating is stopped till the remaining concentrated solution is approximately 4 times to that of compound V by weight; and the remaining concentrated solution is directly used in the next step of the reaction. The high boiling solvent is one, two or more selected from the group consisting of N,N-dimethylformamide, N,N-dimethylacetamide, and dimethyl sulfoxide, and preferably N,N-dimethylacetamide.

As a specific embodiment, in step e), the reaction solvent is one, two or more selected from the group consisting of N,N-dimethylformamide, N,N-dimethylacetamide, and dimethyl sulfoxide, and preferably N,N-dimethylacetamide.

As a specific embodiment, step e) is carried out at a temperature in the range of 10^{~}40°C, and preferably in the range of 20^{~}30°C.

As a specific embodiment, in step e), a condensation agent is added to the reaction, wherein the condensation agent is one, two or more selected from the group consisting of HATU, HBTU, HCTU, PyBOP, and TBTU, and preferably PyBOP.

As a specific embodiment, in step e), an alkaline reagent is added to the reaction, wherein the alkaline reagent is one, two or more organic bases selected from the group consisting of triethylamine, N-methylmorpholine, diisopropylethylamine, and 4-methylaminopyridine, and preferably N-methylmorpholine.

As a specific embodiment, in step e), compound IV and condensation agent are fed at a molar ratio in the range of 1:1.1^{~}1:3; compound IV, compound 9 and the organic base are fed at a molar ratio in the range of 1:1.05:1^{~}1:1.1:3.

As a specific embodiment, the workup mode of step e) is as follows: after the completion of the reaction, ethyl acetate and water are added under stirring to extract, aqueous layer is separated and removed out, and the organic layer is poured into the reaction solvent that is used in the next step and then concentrated till the remaining concentrated solution is approximated 2 times to that of compound IV by weight; and the remaining concentrated solution is directly used in the next step of the reaction without separation.

As a specific embodiment, in step f), the reaction solvent is one, two or more selected from the group consisting of 2-methyltetrahydrofuran, tetrahydrofuran, 1,4-dioxane, acetone, and acetonitrile, and preferably tetrahydrofuran; and the weight amount of the solvent is 4^{~}8 times to that of compound II.

As a specific embodiment, step f) is carried out at a temperature in the range of 10^{~}40°C, and preferably in the range of 15^{~}25°C.

As a specific embodiment, in step f), an alkaline solution is added to the reaction, wherein the alkaline solution is sodium hydroxide solution of 2^{~}4 mol/L, and compound III and sodium hydroxide are fed at a molar ratio in the range of 1:4^{~}1:6.

As a specific embodiment, in step f), an acidic regulator is used, wherein the acidic regulator is hydrochloric acid solution of 1^{~}2 mol/L; and the pH is adjusted to the range of 1^{~}4, and preferably to the range of 2^{~}3.

As a specific embodiment, the workup mode of step f) is as follows: after adjusting the pH, the reaction mixture is stirred to crystalize or stood to crystalize, and preferably stood to crystalize.

As a specific embodiment, in step g), the reaction solvent is a mixture of water and one, two or more selected from the group consisting of acetic acid, tetrahydrofuran, and acetone, and preferably an aqueous tetrahydrofuran solution, and the weight amount of the reaction solvent is 15^{~}20 times to that of compound II.

As a specific embodiment, in step g), during the reaction, a hydrochloric acid solution A is first added and stirred, and subsequently water is added and further stirred, and then a hydrochloric acid solution B is added and stirred to crystalize. The weight amount of hydrochloric acid solution A is 2^{~}3 times to that of compound II, and the weight amount of hydrochloric acid solution B is 6^{~}8 times to that of compound II. The concentrations of hydrochloric acid solutions A and B are 3^{~}6 mol/L, and preferably 4^{~}5 mol/L.

The invention achieves the following beneficial effects: compared with the prior art methods, the method of the invention for preparing (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propanoic acid dihydrochloride has less steps, does not require separation and purification by column chromatography, and is suitable for industrial production. Moreover, in the method of the invention, the six steps for preparing compound II from compound V can be carried out in a continuous feeding mode, do not require purification process; the workup is simple and the loss of the product is minimized; solid product is precipitated from a certain solvent by the salt forming mode of acid and alkaline, to achieve purification effect and meanwhile obtain intermediate II having high purity, and then target compound I is obtained via further salt forming step. The preparation method of the invention has a total yield of more than 35% from compound 1 to the final product compound I, and the purity of the final product is more than 98%. Compared with the prior art (approximate 5% yield), the method of the invention has significantly improved yield and is suitable for large-scale industrial production. The current method had reached a maximum batch size of 50 kg, and showed good process stability and controllable product quality.

### Detailed Description of the Invention

The invention will be further illustrated by combining the following specific examples. The following examples are used to explain the method of the invention and the core concept thereof, and for those skilled in the art, any possible change or substitution without departing from the inventive concept will fall within the protection scope of the invention. In the following examples, where the specific conditions of the experimental methods are not indicated, they are typically the conventional conditions, or are those recommended by the raw material or commodity manufactures; and the solvents without indicating the source are typically conventional solvents that are commercially available. Compounds 1, 2 and 9 were prepared according to the methods described in CN102378574B and WO2007093013 A1, the two patents are hereby incorporated by reference in their entirety.

Prior art invention CN102378574B discloses the method for preparation of (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylpyridin-4-yl)phenyl)propanoic, which utilizes 4-hydroxyacetophenone as the starting material and comprises 14 steps including nucleophilic substitution, bromination, asymmetric reduction, condensation, hydrolysis, etc., the total yield is calculated based on the prior art document which is approximately 5%.

The detection instrument used in the present invention is:
1. NMR
   Instrument model: Bruker DMX-500 nuclear magnetic resonance instrument
2. Mass Spectrometer
   Instrument model: Agilent 6460, test conditions: ESI source

### Example 1: Preparation of compound VII

Compound 1 (83.1 g, 0.2 mol), compound 2 (R is methyl) (75.9 g, 0.3 mol), toluene (300 g) and DMAP (48.9 g, 0.4 mol) were charged into a reaction flask, and stirred at 20°C for 16 hours. TLC was used to monitor the time point when compound 1 disappeared. After the completion of the reaction, a 5% aqueous ammonium chloride solution (820 g) was added. The aqueous layer was separated and removed out, and the organic layer was washed with saturated brine and concentrated. Methanol/water (500 g/500 g) was further added to the concentrated solution and stirred to crystalize. The precipitated crystals were filtered and dried to give 95.2 g compound VII, with a yield of 88%. ¹H NMR (500 MHz, d₆-DMSO) δ 9.54 (s, 1H), 7.72 (d, J =1.5Hz, 1H), 7.66 (d, J = 8.5Hz, 1H), 7.46-7.41 (m, 3H), 7.29(d, J =2Hz, 1H), 7.18(d, J =1.5Hz, 1H), 7.14 (s, 1H), 7.07(d, J =8.5Hz, 2H), 6.99(d, J =8.5Hz, 1H), 5.24-5.33 (m, 1H), 5.15 (s, 2H), 4.42-4.40 (m, 1H), 4.01 (s, 5H), 4.17 - 4.10 (m, 3H), 2.00 (s, 3H), 1.23 (t, J =7.0Hz, 3H); ¹³C NMR (100 MHz, CDCl3) δ 169.3, 165.1, 158.2, 144.8, 142.5, 138.2, 131.1(2C), 130.7, 130.4, 129.4, 128.7, 128.3, 127.8, 127.7, 126.8, 125.1, 124.1, 118.0, 117.3, 114.9, 74.3, 68.0, 67.6, 60.6, 22.4, 14.1; MS (ESI) m/z (%): 542.1 (100) [M+H].

### Example 2: Preparation of compound VI

Compound VII (54.2 g, 0.1 mol) and N-methylpyrrolidone (542 g) were charged into a reaction flask and stirred to dissolve, and then hydrochloric acid of 6 mol/L (220.0 g, 1.2 mol) was added. The reaction mixture was heated to 85°C to react for 18 hours, and TLC was used to monitor the time point when compound VII disappeared. After the completion of the reaction, the reaction mixture was cooled down, and water (542 g) was added and stirred to crystalize. After filtration, wet product was obtained, and then was slurried with methanol/dichloromethane (300 g/300 g). After filtration and drying, 40.1 g compound VI was obtained, with a yield of 85%. ¹H NMR (500 MHz, d₆-DMSO) δ 13.11 (s, 1H), 9.06 (s, 1H), 7.72 (d, J = 1.5Hz, 1H), 7.65 (d, J = 8.5Hz, 4H), 7.47-7.41 (m, 4H), 7.25 (dd, J = 3.5, 2.0Hz, 2H), 7.06 (d, J = 9.0Hz, 2H), 6.92 (d, J = 8.5Hz, 1H), 6.37 (s, 1H), 5.18 (d, J = 2.0 Hz, 1H), 5.14 (s, 2H), 4.39 - 4.37 (m, 1H), 4.11 - 4.07 (m, 1H); 13C NMR (100 MHz, d₆-DMSO) δ 166.4, 158.1, 143.0, 142.4, 140.5, 138.3, 131.1, 130.7, 130.4, 129.4, 128.9, 128.5, 128.3(2C), 127.7, 123.1, 117.4, 117.0, 114.8(2C), 109.6, 74.1, 68.1, 67.6; MS (ESI) m/z (%): 471.0 (100) [M-H]; HRMS (ESI) Calcd. for C₂₄H₁₉Cl₂O₆ 473.0391 [M+H], found: 473.0391.

### Example 3a: Preparation of compound V

Compound VI (23.6 g, 0.05 mol), and toluene (142 g) were charged into a reaction flask, the temperature was maintained at 25°C or below 25°C, and then isopropylamine (5.9 g, 0.1 mol) and (S)-phenylpropylamine (10.8 g, 0.08 mol) were successively added and stirred. Sodium borohydride (3.7 g, 0.1 mol) and 2-ethylpentanic acid (39.1g, 0.3mol) were mixed in toluene/dimethylacetamide to form reducing agent solution, and then were added to the reaction system at 15^{~}20°C. After the completion of the reaction, the reaction mixture was concentrated under reduced pressure. The residue was dissolved in dimethylacetamide. After cooling down, the solution was subject to precipitation in methanol. After filtration, washing and drying, 20.2 g compound V was obtained, with a yield of 68.2%. ¹H NMR (500 MHz, d₆-DMSO) δ 7.53(s, 1H), 7.49-7.41 (m, 4H), 7.32 (d, J =8.5Hz, 2H), 7.27-7.24 (m, 1H), 7.21 (d, J = 8.5Hz, 2H), 6.99 (d, J = 8.5Hz, 2H), 6.82 (d, J = 8.0 Hz, 1H), 6.50 (s, 1H), 6.43 (d, J = 8.0Hz, 1H), 5.03 (s, 2H), 4.99 (d, J = 8.0 Hz, 1H), 4.29 (d, J = 2.0 Hz, 1H), 4.27-4.16 (m, 1H), 3.96-3.92 (m, 1H), 3.83-3.83 (m, 1H), 3.00-2.96 (m, 2H), 2.24-2.17 (m, 1H), 2.06-1.99 (m, 1H), 0.81 (t, J = 7.0 Hz, 3H), MS (ESI) m/z (%): 592.1 (100) [M+H].

### Example 3b: Preparation of compound V

Compound VI (23.6 g, 0.05 mol), and toluene (142 g) were charged into a reaction flask, the temperature was maintained at 25°C or below 25°C, and then triethylamine (5.6g, 0.055mol) and (S)-phenylpropylamine (8.95g, 0.06mol) were successively added and stirred. Sodium borohydride (3.7 g, 0.1 mol) and 2-ethylhexanoic acid (32.4g, 0.225mol) were mixed in toluene/dioxane to form reducing agent solution, and then were added to the reaction system at 15^{~}20°C. After the completion of the reaction, the reaction mixture was concentrated under reduced pressure. The residue was dissolved in dimethylformamide. After cooling down, the solution was subject to precipitation in methanol. After filtration, washing and drying, 19.8 g compound V was obtained, with a yield of 66.8%. The characterization data of the obtained compound is the same as that of Example 3a.

### Example 4: Preparation of compound IV

Compound V (59.2 g, 0.1 mol), paraformaldehyde (14.8 g), trifluoromethanesulfonic acid (118 mL), chlorobenzene (592 g) and isopropyl acetate (177.6 g) were charged into a reaction flask, stirred and mixed homogenously, and reacted at 35°C for 3^{~}5 hours. After the completion of the reaction, the reaction mixture was cooled down to below 0°C, sodium hydroxide solution of 2 mol/L (651 g) was added and then stirred for 10 minutes. Aqueous phase was separated, and organic phase was washed twice with saturated brine. N,N-dimethylacetamide was added to the organic phase, and the organic phase was concentrated to approximately a 118 g solution of compound IV, which was directly used in the next step of the reaction, with the yield being recorded as 100%. 1H NMR (500 MHz, d6-DMSO) δ 7.72 (d, J = 2.0Hz, 1H), 7.66 (d, J = 8.5Hz, 1H), 7.46-7.44 (m, 1H), 7.41-7.34 (m, 4H), 7.29-7.26 (m, 3H), 7.06 (d, J = 8.5Hz, 2H), 6.65 (d, J = 23Hz, 2H), 5.15 (s, 1H), 5.13-5.10 (m, 1H), 4.32-4.29 (m, 1H), 4.07-3.97 (m, 3H), 3.93-3.90 (m, 1H), 3.42 (t, J=4.0 Hz, 1H), 2.80 (s, 1H), 2.10-2.03 (m, 1H), 1.65-1.59 (m, 1H), 0.59 (t, J = 7.0 Hz, 3H); 13C NMR (100 MHz, d6-DMSO) δ 174.1, 158.6, 142.4, 142.3, 141.4, 138.8, 131.6, 131.2, 130.8, 129.9, 129.7, 128.8(2C), 128.7(2C), 128.7(2C), 128.2, 128.0, 127.7, 126.0, 116.3, 115.3(2C), 114.9, 74.5, 68.7, 68.1, 56.4, 47.4, 31.7, 25.6, 10.9; MS (ESI) m/z (%): 604.1 (100) [M]+

### Example 5: the preparation of compound III

First, compound 9' was pretreated to give compound 9, according to the following dissociation scheme:

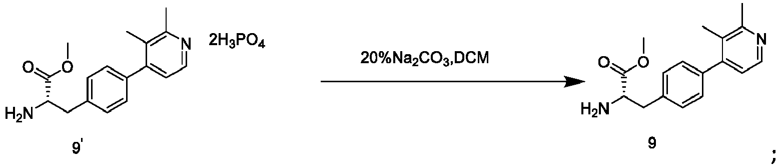

Compound 9' was dissolved in water, and after dissolution, a 20% sodium carbonate solution was added to adjust pH to 8^{~}9, and then dichloromethane was added to extract three times. The organic phase was combined and concentrated, and then DMA was added for entrainment and further concentrated to remove out dichloromethane. The concentrate was ready for use, with the yield being estimated as 80^{~}90%;

A solution of compound IV (59.2 g, 0.1 mol) and a solution of compound 9 (34.1 g, 0.12mol) in DMA were mixed in a reaction flask, NMM (15.2 g, 0.15 mol) was added, and then condensation agent PyBop (67.7 g, 0.13 mol) was added. The reaction mixture was stirred at 25°C for 4^{~}5 hours. After the completion of the reaction, ethyl acetate was added, and then purified water was added. The reaction mixture was stirred and extracted for 10 minutes, and then separated the phases. Tetrahydrofuran was added to the organic phase, and then ethyl acetate was removed out by concentration. The remaining concentrated solution was approximately 118 g, and was directly used in the next step of the reaction, with the yield being recorded as 100%. ¹H NMR (500 MHz, d6-DMSO) δ 8.27 (d, J=5.0Hz, 1H), 8.00 (d, J = 8.0Hz, 1H), 7.72 (d, J = 1.5Hz, 1H), 7.66 (d, J =8.0Hz, 1H), 7.45-7.43 (m, 1H), 7.33-7.30 (m, 2H), 7.27-7.19 (m, 7H), 7.98-6.91 (m, 5H), 6.68 (s, 1H), 6.53 (s, 1H), 5.12 (s, 1H), 5.10-5.08 (m, 1H), 4.73-4.69 (m, 1H), 4.30-4.27 (m, 1H), 3.84-3.80 (m, 1H), 3.67 (s, 3H), 3.57-3.55(m, 1H), 3.51-3.43 (m, 1H), 3.25-3.23 (m, 1H), 3.11-3.06 (m, 1H), 2.95-2.91 (m, 1H), 2.83-2.79 (m, 1H), 2.71-2.67 (m, 1H), 2.46 (s, 1H), 2.08 (s, 1H), 1.92-1.86 (m, 1H), 1.70-1.64 (m, 1H), 0.55 (t, J = 7.0 Hz, 3H); ¹³C NMR (100 MHz, d6-DMSO) δ 173.4, 171.9, 158.5, 157.8, 148.9, 145.9, 142.0, 141.8, 140.9, 138.7, 138.0, 136.6, 131.6, 131.2, 130.9, 129.9, 129.8, 129.6(2C), 129.5, 129.1(2C), 129.0(2C), 128.8, 128.7(2C), 128.5, 128.2(2C), 127.7, 122.6, 115.9, 115.2(2C), 114.9, 74.3, 68.7, 68.2, 68.1, 59.0, 52.7, 52.5, 46.6, 37.0, 29.3, 26.0, 23.6, 16.2, 11.2; MS (ESI) m/z (%): 870.2 (100) [M]+

### Example 6: Preparation of compound I

The concentrated solution of compound III (62.6 g, 0.1 mol) in tetrahydrofuran, and tetrahydrofuran (250.4 g) were charged into a reaction flask, stirred and mixed homogenously. A sodium hydroxide solution of 2 mol/L (sodium hydroxide: 16 g) was added, and the reaction mixture was reacted at 30°C for 18^{~}20 hours. After the completion of the reaction, a hydrochloric acid solution of 1.5 mol/L was added to adjust the pH to about 2. The pH remained unchanged after repeated measurement, and then stopped stirring. The reaction mixture was stood to crystalize for 6 hours, and then 66.1 g compound II was obtained after filtration, with a yield of 74% and purity of 98.3%.

Compound II (44.6 g, 0.05 mol), tetrahydrofuran (133.8 g), and water (1338 g) was charged into a reaction flask, and then a hydrochloric acid solution of 4 mol/L (89.2 g) was added and stirred until the system was completely clear. Then purified water (802.8 g) was added and stirred for 1 hour, and subsequently a hydrochloric acid solution of 4mol/L (267.6 g) was added and stirred for 2 hours to crystalize. 45.4 g target compound I was obtained after filtration and drying, with a yield of 98% and purity of 98.3%. ¹H NMR (500 MHz, d5-Pyridine) δ 8.83 (d, J=8.0 Hz, 1H), 8.61 (dd, J=5.0, 1.5 Hz, 1H), 7.75 (s, 1H), 7.62 (s, 1H), 7.55-7.50 (m, 3H), 7.45 (d, J=8.0 Hz, 1H), 7.40-7.30 m, 7H), 7.30 (d, J = 8.0 Hz, 2H), 7.12 (dd, J=14.5, 6.5 Hz, 3H), 7.03 (s, 1H), 6.98 (s, 1H), 5.49-5.46 (m, 1H), 5.22 (d, J=9.0 Hz, 1H), 5.19 (s, 2H), 4.40 (d, J=11.0 Hz, 1H), 4.09 (d, J=5.0 Hz, 1H), 4.06-3.97 (m, 2H), 3.87 (dd, J = 10.0, 5.0 Hz, 1H), 3.72 (d, J =14.0 Hz, 1H), 3.54-3.50 (m, 1H), 3.49-3.42 (m, 2H), 3.12 (dd, J = 15.0, 7.5 Hz, 1H), 2.64 (s, 3H), 2.20-2.13 (m, 1H), 2.10 (s, 3H), 2.07-2.00 (m, 1H), 0.69 (t, J=7.5 Hz, 3H); ¹³C NMR (100 MHz, d5-Pyridine) δ 174.7, 174.3, 159.7, 157.5, 151.9, 144.6, 143.4, 143.2, 141.3, 139.0, 138.6, 138.2, 133.1, 132.2, 131.6(2C), 131.0, 130.8(2C), 130.6, 130.5(2C), 130.3, 130.0, 129.8(2C), 129.5, 129.1(2C), 128.7, 128.5, 128.2(2C), 117.0, 116.1, 116.0(2C), 75.6, 70.6, 70.0, 69.2, 60.9, 54.3, 48.5, 38.7, 31.1, 27.2, 22.7, 16.7, 11.8; HRMS (ESI) Calcd. For C₅₀H₄₇Cl₂N₃O₆ 856.2915 [M+H], found: 856.2905. The chloride ion content measured by titration method is 7.02%, proves that it contains two molecules of hydrochloric acid, that the molecular formula is C₅₀H₄₇Cl₂N₃O₆.2HCl.

### Example 7: Batch enlargement experiment

In accordance with the methods of Examples 4-6, compound I was synthesized from compound V. The results were shown in Table 1.

**Table 1 Results of the batch enlargement experiment**

| Batch | Fed amount of compound V (kg) | Purity of compound II (%) | Yield of compound I (%)* | Purity of compound I (%) |
|---|---|---|---|---|
| 2 | 1 | 98.2 | 75.7 | 98.2 |
| 3 | 2.5 | 98.0 | 76.0 | 98.0 |
| 4 | 15 | 98.2 | 77.3 | 98.2 |
| 5 | 45 | 97.9 | 80.1 | 98.1 |

| | | | | |
|---|---|---|---|---|
| *note: the yield was the total yield of the four steps. | | | | |

The characterization data of the obtained compound are the same as those in Example 6.

## Claims

1. A method for preparing (S)-2-(3S,8S)-3-(4-(3,4-dichlorobenzyloxy)phenyl-7-((S)-1-phenylpropyl)-2,3,6,7,8,9-hexahydro-[1,4]-dioxino[2,3-g]isoquinolin-8-ylformylamino)-3-(4-(2,3-dimethylp yridin-4-yl)phenyl)propanoic acid dihydrochloride, **characterized in that**, the method comprises the following steps:
step a): reacting compound 1 with compound 2 via addition reaction to give compound VII;
step b): subjecting compound VII to hydrolysis to give compound VI;
step c): reacting compound VI with S-phenylpropylamine via ammoniation reduction to give compound V;
with the following scheme: 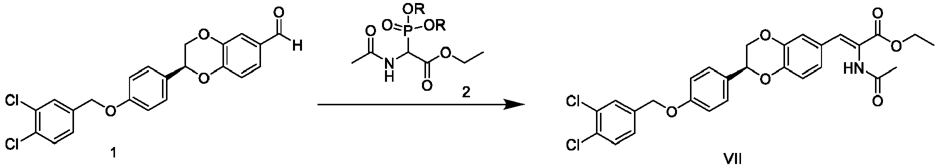 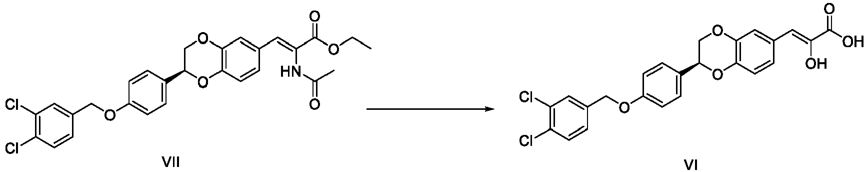 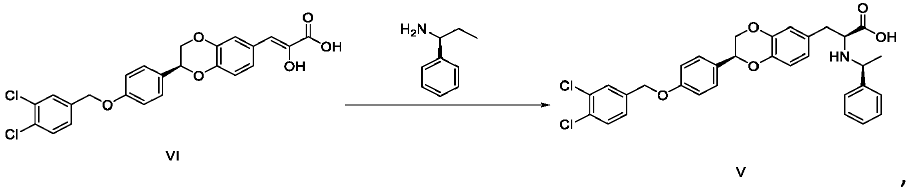
wherein the reaction step c) is carried out under alkaline condition using organic base as the alkaline reagent, wherein the organic base is triethylamine or/and isopropylamine.

2. The method according to claim 1, **characterized in that**, the method further comprises the
following steps:
step d): reacting compound V with paraformaldehyde via cyclization to give compound IV;
step e): reacting compound IV with compound 9 via condensation to give compound III;
step f): subjecting compound III to hydrolysis under alkaline condition, and then adjusting pH to
acidic condition, to give compound II,
step g): reacting compound II with hydrochloric acid solution to give compound I;
with the following scheme:

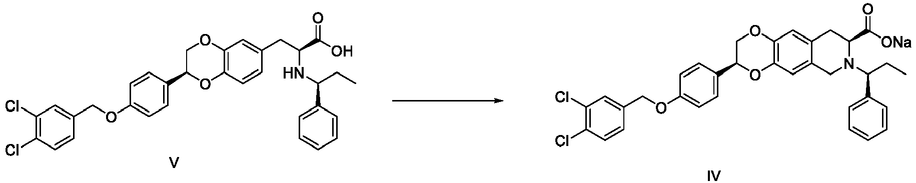

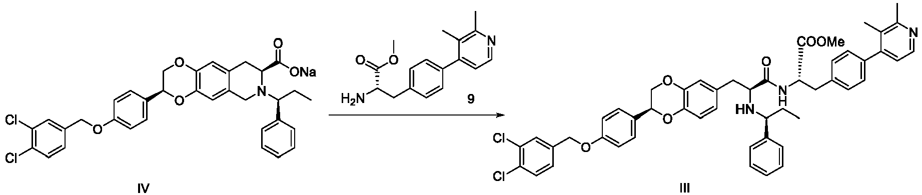

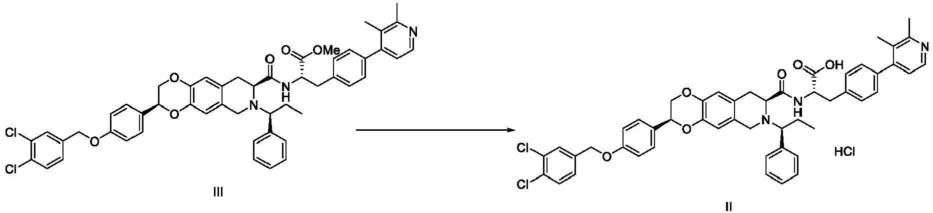

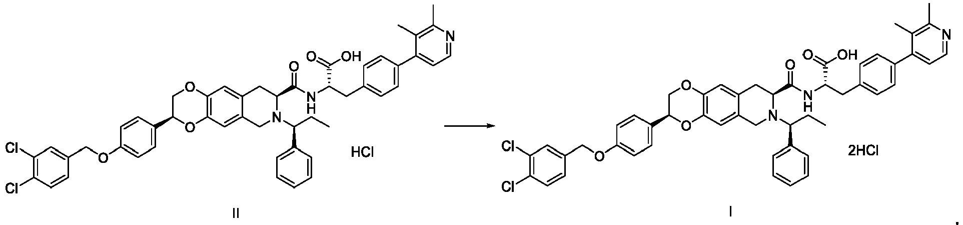

3. The method according to claim 1 or 2, **characterized in that**, in step a), the reaction is carried out under alkaline condition using organic base as the alkaline reagent, wherein the organic base is one, two or more selected from the group consisting of DBU, DMAP, triethylamine, isopropylamine, dibutylamine, piperazine, piperidine, and morpholine, and preferably DMAP;
and/or the reaction solvent is one, two or more selected from the group consisting of dichloromethane, toluene, tetrahydrofuran, and 2-methyltetrahydrofuran, and preferably 2-methyltetrahydrofuran;
and/or compound 1, compound 2 and the alkaline reagent are fed at a molar ratio in the range of 1:1.05:1.5^{~}1:2:3; and/or the R group of Compound 2 is selected from C1^{~}C10 alkane, C3^{~}C8 cycloalkane, benzyl, aryl and heteroaryl, preferably methyl.

4. The method according to claim 1 or 2, **characterized in that**, in step b), the reaction solvent is one, two or more selected from the group consisting of 1,4-dioxane, N-methylpyrrolidone, dimethyl sulfoxide, dimethylacetamide and dimethylformamide, and preferably N-methylpyrrolidone;
and/or the reaction is carried out under acidic condition using acidic aqueous solution as the acidic reagent, wherein the acid is one, two or more selected from the group consisting of hydrochloric acid, sulfuric acid, and phosphoric acid, and preferably hydrochloric acid;
and/or compound VII and the acidic aqueous solution are fed at a molar ratio in the range of 1:10^{~}1:13, and the concentration of the acidic aqueous solution is 2mol/L^{~}6mol/L.

5. The method according to claim 1 or 2, **characterized in that**, in step c), the reaction solvent is one, two or more selected from the group consisting of dioxane, N-methylpyrrolidone, dimethyl sulfoxide, toluene, dimethylformamide, and dimethylacetamide, and preferably a mixture of toluene and dimethylacetamide;
and/or a reducing agent is added to the reaction, where the reducing agent is NaBH(OCOR)₃ with R being C₆^{~}C₁₂ alkyl and preferably C₇-C₉ alkyl;
and/or the molar amount of the reducing agent is 1^{~}3 times to that of compound VI, and preferably 1.5^{~}2 times;
and/or the compound VI, S-phenylpropylamine and the organic base are fed at a molar ratio in the range of 1:1.05:1.05^{~}1:1.1:3.

6. The method according to claim 2, **characterized in that**, in step d), the reaction solvent is one, two or more selected from the group consisting of toluene, xylene, chlorobenzene, ethyl acetate, isopropyl acetate, propyl acetate, and butyl acetate, and preferably a mixture of chlorobenzene and isopropyl acetate;
and/or acid catalyst is added to the reaction, where the acid catalyst is one, two or more selected from the group consisting of trifluoroacetic acid, concentrated sulfuric acid, hydrochloric acid, phosphoric acid, and trifluoromethanesulfonic acid, and preferably trifluoromethanesulfonic acid;
and/or the reaction is carried out at a temperature in the range of 20^{~}50°C, and preferably in the range of 30^{~}40°C.

7. The method according to claim 2, **characterized in that**, in step e), the reaction solvent is one, two or more selected from the group consisting of N,N-dimethylformamide, N,N-dimethylacetamide, and dimethyl sulfoxide, and preferably N,N-dimethylacetamide;
and/or a condensation agent is added to the reaction, where the condensation agent is one, two or more selected from the group consisting of HATU, HBTU, HCTU, PyBOP, and TBTU, and preferably PyBOP;
and/or an alkaline reagent is added to the reaction, wherein the alkaline reagent is one, two or more organic bases selected from the group consisting of triethylamine, N-methylmorpholine, diisopropylethylamine, and 4-methylaminopyridine, and preferably N-methylmorpholine;
and/or compound IV and the condensation agent are fed at a molar ratio in the range of 1:1.1^{~}1:3;
and/or compound IV, compound 9 and the organic base are fed at a molar ratio in the range of 1:1.05:1^{~}1:1.1:3;
and/or the reaction is carried out at a temperature in the range of 10^{~}40°C, and preferably in the range of 20^{~}30°C.

8. The method according to claim 2, **characterized in that**, in step f), the reaction solvent is one, two or more selected from the group consisting of 2-methyltetrahydrofuran, tetrahydrofuran, 1,4-dioxane, acetone, and acetonitrile, and preferably tetrahydrofuran;
and/or the weight amount of the solvent is 4^{~}8 times to that of compound II; the reaction is carried out at a temperature in the range of 10^{~}40°C, and preferably in the range of 15^{~}25°C;
and/or alkaline solution is added to the reaction, wherein the alkaline solution is a sodium hydroxide solution of 2^{~}4mol/L, and compound III and sodium hydroxide are fed at a molar ratio in the range of 1:4^{~}1:6;
and/or acidic regulator is used in the reaction, with the acidic regulator being hydrochloric acid solution of 1^{~}2mol/L;
and/or the pH is adjusted to the range of 1^{~}4, and preferably pH is 2^{~}3.

9. The method according to claim 2, **characterized in that**, in step g), the reaction solvent is a mixture of water and one, two or more selected from the group consisting of acetic acid, tetrahydrofuran, and acetone, and preferably an aqueous tetrahydrofuran solution;
and/or the weight amount of the reaction solvent is 15^{~}20 times to that of compound II; and/or during the reaction, a hydrochloric acid solution A is first added and stirred, and subsequently water is added and further stirred, and then a hydrochloric acid solution B is added and stirred to crystalize;
and/or the weight amount of hydrochloric acid solution A is 2^{~}3 times to that of compound II, and the weight amount of hydrochloric acid solution B is 6^{~}8 times to that of compound II;
and/or the concentrations of hydrochloric acid solutions A and B are 3^{~}6 mol/L, and preferably 4^{~}5 mol/L.

10. A method for preparing compound V, **characterized in that**, the method comprises the following steps:
a) 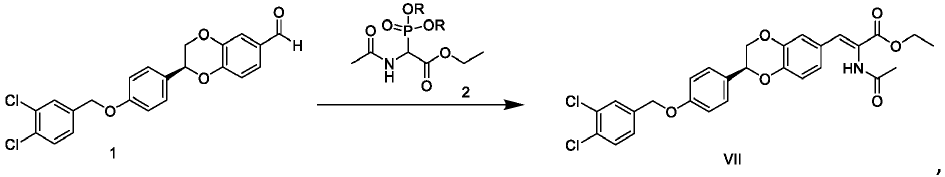
b) 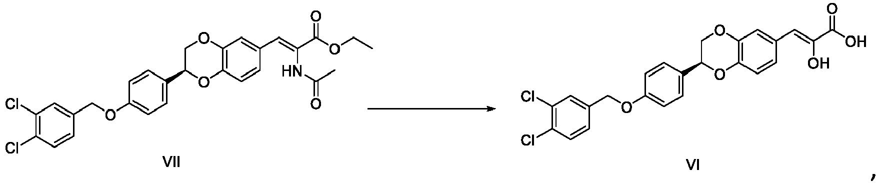
c) 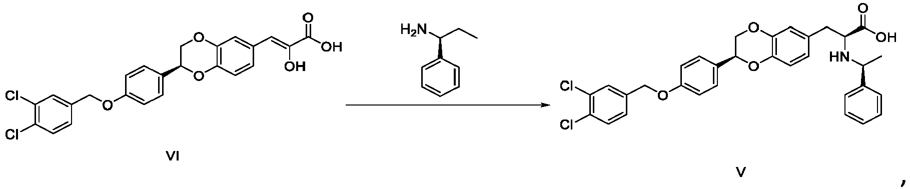 wherein the reaction step c) is carried out under alkaline condition using organic base as the alkaline reagent, wherein the organic base is triethylamine or/and isopropylamine.
